# EUROPÄISCHE PATENTANMELDUNG

(11) **EP 2 289 520 A1**
(43) Veröffentlichungstag der Anmeldung: **02.03.2011**
(21) Anmeldenummer: 10011214.3
(22) Anmeldetag: 02.01.2004
(51) Int. Cl.: A61K 31/60, A61P 31/16

(54) **Verwendung von Acetylsalicylsäure zur Prophylaxe und/oder Therapie von Influenzavirus Infekten.**

(30) Priorität: 03.01.2003 DE 10300222
(62) Teilanmeldung aus: 04700037.7
(71) Anmelder: Activaero GmbH, 35285 Gemünden (DE)
(72) Erfinder: Planz, Oliver, 72108 Rottenburg (DE); Pleschka, Stephan, 35390 Giessen (DE); Sedlacek, Hans-Harald, 35041 Marburg (DE); Ludwig, Stephan, 48149 Münster (DE)
(74) Vertreter: Vossius & Partner

(57) **Zusammenfassung**

Die vorliegende Erfindung betrifft die Verwendung bevorzugt mindestens einer Wirksubstanz zur Prophylaxe und/oder Therapie einer Viruserkrankung, wobei diese Wirksubstanz mindestens eine Komponente des zellulären Signalübertragungsweges zur Aktivierung des Transkriptionsfaktors NF-kB derart hemmt, dass die Virusvermehrung gehemmt wird. Die vorliegende Erfindung betrifft des weiteren die lokale, bevorzugt die aerogene Verabreichung des erfindungsgemässen Wirkstoffes zur Hemmung einer Virusvermehrung. Die erfindungsgemässe Wirksubstanz kann mit mindestens einer weiteren antiviral wirksamen Substanz zur Prophylaxe und/oder Therapie einer Viruserkrankung kombiniert werden.

## Beschreibung

### Gebiet der Erfindung.

Die Erfindung betrifft ein Testsystem zur Findung von Wirksubstanzen, welche zur Prophylaxe und/oder Behandlung von Viruserkrankungen geeignet sind, Verwendungen solcher Wirksubstanzen zur Herstellung einer pharmazeutischen Zusammensetzung zur Prophylaxe und/oder Behandlung von Viruserkrankungen, Formulierungen für solche pharmazeutischen Zusammensetzungen sowie Verfahren zur Herstellung solcher pharmazeutischen Zusammensetzungen.

### Stand der Technik und Hintergrund der Erfindung

Infektionen mit RNA- oder DNA-Viren stellen eine bedeutende Gesundheitsbedrohung für Mensch und Tier dar. Zu den RNA-Viren gehören die Negativ-Strang-RNA Viren, wie beispielsweise Influenza Viren oder das Borna-Disease Virus. Infektionen mit Influenza-Viren gehören immer noch zu den großen Seuchen der Menschheit und fordern Jahr für Jahr eine Vielzahl an Todesopfern. Sie sind gesamtwirtschaftlich, etwa durch krankheitsbedingte Arbeitsunfähigkeit, ein immenser Kostenfaktor. Von ebenfalls wichtiger wirtschaftlicher Bedeutung sind Infektionen mit dem Borna Disease Virus (BDV), das vor allem Pferde und Schafe befällt, jedoch auch schon beim Menschen isoliert wurde und hier in Zusammenhang mit neurologischen Erkrankungen gebracht wird.

Das Problem der Bekämpfung von insbesondere RNA-Viren ist die durch eine hohe Fehlerrate der viralen Polymerasen verursachte Wandlungsfähigkeit der Viren, die sowohl die Herstellung geeigneter Impfstoffe als auch das Entwickeln von antiviralen Substanzen sehr schwierig macht.

Darüber hinaus hat sich gezeigt, dass die Anwendung von antiviralen Substanzen, die direkt gegen Funktionen des Virus gerichtet sind, zwar zu Therapiebeginn eine gute antivirale Wirkung zeigen, aber mutationsbedingt sehr schnell zur Selektion resistenter Varianten führt. Ein Beispiel hierfür ist das anti-Influenza-Agens Amantadin und dessen Derivate, welches bzw. welche gegen ein Transmembranprotein des Virus gerichtet ist bzw. sind. Innerhalb kurzer Zeit nach Anwendung bilden sich resistente Varianten des Virus.

Ein weiteres Beispiel sind die neuen Therapeutika für Influenzainfektionen, die das Influenza-virale Oberflächenprotein Neuraminidase hemmen. Hierzu gehört beispielsweise Relanza. In Patienten wurden bereits Relanza-resistente Varianten gefunden (Gubareva et al., J Infect Dis 178, 1257-1262, 1998). Die Hoffnungen, die in dieses Therapeutikum gelegt wurden, konnten somit nicht erfüllt werden.

Aufgrund ihrer meist sehr kleinen Genome und damit verbundener begrenzter Kodierungskapazität für replikationsnotwendige Funktionen sind alle Viren in starkem Maße auf Funktionen ihrer Wirtszellen angewiesen. Durch Einflussnahme auf solche zelluläre Funktionen, die für die virale Replikation notwendig sind, ist es möglich, die Virüsreplikation in der infizierten Zelle negativ zu beeinträchtigen. Hierbei besteht für das Virus nicht die Möglichkeit, durch Anpassung, insbesondere durch Mutationen, die fehlende zelluläre Funktion zu ersetzen, um so dem Selektionsdruck zu entweichen. Dies konnte am Beispiel des Influenza A Virus mit relativ unspezifischen Hemmstoffen gegen zelluläre Kinasen und Methyltransferasen bereits gezeigt werden (Scholtissek und Müller, Arch Virol 119, 111-118, 1991).

Es ist bekannt, dass Zellen über eine Vielzahl von Signalübertragungswegen verfügen, mit Hilfe derer auf die Zelle einwirkende Signale in den Zellkern übertragen werden. Dadurch kann die Zelle auf äußere Reize reagieren und mit Zellproliferation, Zellaktivierung, Differenzierung oder kontrolliertem Zelltod reagieren.

Diesen Signalübertragungswegen ist gemeinsam, dass sie mindestens eine Kinase enthalten, welche durch Phosphorylierung mindestens ein nachfolgend signalübertragendes Protein aktivieren.

Bei Betrachtung der zellulären Prozesse, die nach Virusinfektionen induziert werden, findet man, dass eine Vielzahl von DNA-und RNA-Viren in der infizierten Wirtszelle bevorzugt einen definierten Signalübertragungsweg, den sogenannte Raf/MEK/ERK-Kinase-Signalübertragungsweg aktivieren. Dieser Signalübertragungsweg ist einer der wichtigsten Signalübertragungswege in einer Zelle und spielt eine bedeutende Rolle in Proliferations-und Differenzierungsprozessen (Cohen, Trends in Cell Biol 7, 353-361, 1997; Robinson und Cobb, Curr.Opin.Cell Biol 9, 180-186, 1997; Treisman, Curr.Opin Cell Biol 8, 205-215, 1996).

Die Untersuchung der Rolle dieses Signalübertragungsweges in zellulären Entscheidungsprozessen hat zur Identifizierung mehrrer pharmakologischer Inhibitoren geführt, welche den Signalübertragungsweg unter anderem auf der Ebene von MEK, also relativ am Beginn des Signalübertragungsweges hemmen (Alessi et al., J Biol Chem 270, 27489-27494, 1995; Cohen, Trends in Cell Biol 7, 353-361, 1997; Dudley et al., PNAS USA 92, 7686-7689, 1995; Favata et al., J Biol Chem 273, 18623-18632, 1998).

Neuere Daten zeigen, dass die Inhibition des Ras-Raf-MEK-ERK-Signalübertragungsweges oder eines weiteren Signalübertragungsweges, des MEKK/SEK/JNK-Signalübertragungsweges, durch Wirksubstanzen, welche relativ selektiv eine der an diesem Signalübertragungsweg beteiligten Kinasen, beispielsweise die MEK oder die SEK inhibieren, die intrazelluläre Vermehrung von intranukleär replizierenden Negativstrang-Viren, beispielsweise von Influenza A Virus und Borna Disease Virus (BDV) drastisch hemmen kann (Pleschka et al., Nature Cell Biol 3, 301-305, 2001; Planz et al., J Virol 10, 4871-4877, 2001; PCT/DE 01/01292; DE 101 38 912).

Bislang war bekannt, dass Influenza- Viren vorzugsweise den Raf-MEK-ERK-Signalübertragungsweg oder den MEKK/SEK Signalübertragungsweg für ihre Vermehrung nutzen und demzufolge eine Hemmung dieser Signalübertragungswege zu einer vollständigen Hemmung der Virusvermehrung führt. Da in einer Zelle die Signalübertragungswege jedoch kaum eine in sich abgeschlossene Funktionen aufweisen, sondern bei Aktivierung des einen Signalübertragungsweges über Kreuzvernetzungen weitere Signalübertragungswege in unterschiedlichem Ausmaß zusätzlich aktiviert werden, ist grundsätzlich nicht auszuschließen, dass ein gehemmter Signalübertragungsweg sowohl von der Zelle als auch von den Viren umgangen werden kann und der therapeutische Effekt einer Wirksubstanz, die eine Virusvermehrung durch Hemmung eines bestimmten Signal-übertragungsweges hemmt, hierdurch eingeschränkt werden könnte.

Daher besteht ein großer Bedarf für das Auffinden und Verwenden antiviraler Wirksubstanzen, welche zusätzlich oder ergänzend zu solchen wirken, die Kinasen von zellulären Signalübertragungswegen, beispielsweise des Raf-MEK-ERK-Signalübertragungsweges oder des MEKK/SEK/JNK-Signalübertragungsweges hemmen. Solche Wirkstoffe sind bereits in den Literaturstellen PCT/DE 01/01292 und DE 101 38 912 beschrieben worden.

Einer der bedeutenden Signalübertragungswege in der Zelle ist der nukleäre Faktor von kappaB (NF-kB)-Signalübertragungs- weg. Zentraler Bestandteil dieses Übertragungsweges ist der heterodimere NF-kB-Transkriptions-Proteinkomplex, bestehend einerseits aus p50 [entstanden aus der Proteolyse von NF-kB1 (p105)] oder aus p52 [entstanden aus der Proteolyse von NF-kappaB2 (p100)] und andererseits aus p65 (RELA), c-REL oder RELB. Der häufigste NF-kB-Komplex besteht aus p50 und p65.
Die Transkriptionsaktivität dieses NF-kB-Komplexes wird gehemmt durch die Bindung der Inhibitorproteine von NFkB (IkB) an die nukleäre Bindesequenz von p65. Hierdurch verbleibt der Komplex im Zytoplasma. Aktivierung der Zelle beispielsweise durch Wachstumsfaktoren, Chemokine, TNFalpha, Il-1, CD40-ligand, LPS oder im Rahmen einer Infektion mit Viren führt zur Aktivierung von Kinasen wie der "NF-kB-induzierenden Kinase" (NIK), der Kinase TAK, der Kinase AKT und möglicherweise auch der Kinase MEKK1. Diese Kinase führen zur Aktivierung des "Inhibitor von kB (IkB)-Kinase" (IKK)-Komplexes, bestehend aus IKKalpha, IKKbeta und NEMO. Aktiviertes IKK phosphoryliert IkB-und führt so zu dessen Degradierung und erlaubt damit die Freisetzung, nukleäre Translokation und Aktivierung der Transskriptionsaktivität des p50/p65- Heterodimers. Ein weiterer NF-kB Komplex besteht aus p100 und RELB. Aktivierung der Zelle durch Lymphotoxine führt, vermutlich bevorzugt vermittelt durch NIK, zur Aktivierung von IKKalpha. Das aktivierte IKK induziert die Phosphorylierung und damit die Proteolyse von p100 zum p52, welches wiederum im Komplex mit dem RELB in den Zellkern translozieren und dort als Transkriptionsfaktor aktiv werden kann.

Es ist bekannt, dass i) nicht-steroidale anti-inflammatorische Substanzen (NSAIDs), wie beispielsweise Sulindac (Yamamoto et al., J Biol Chem 274,27307-27314, 1999; Berman et al., Clin Cancer Res 8, 354-360, 2002) oder Derivate von Sulindac wie beispielsweise Sulindac Sulfoxid, Sulidac Sulfon, Sulindac Sulfid oder Benzylamid Sulindac-Analoga (Moon und Lerner, Cancer Research 62, 5711-5719,2002) oder Acetylsalizylsäure oder Salizylsäure (Yin et al., Nature 396, 77-80,1998) oder Curcumin (Oncogene 18, 6013-6020, 1999) durch Hemmung der IKKbeta, ii) NEMO bindende Peptide (May et al., Science 289, 1550-1554, 2000), Proteosom Inhibitoren wie beispielsweise PS-341 (Tan und Waldmann, Cancer Res 62, 1083-1086,2002; Adams Trends Mol Med 8, 49-54, 2002) oder iii) Antisense Nukleotidsequenzen spezifisch für p65 oder p50 (Higgins et al., PNAS-USA 90, 9901-9905,1993) die Aktivierung des NF-kB-Signalübertragungsweges hemmen können. Bislang wurden diese Hemmstoffe jedoch ausnahmslos geprüft für ihre Verwendung als Wirkstoffe zur Beeinflussung von Entzündungen und des Wachstums von Tumoren, da bei beiden Indikationen die beteiligten Zellen eine erhöhte Aktivierung des NF-kB Signalübertragungsweg aufweisen (Karin et al., Nature Reviews Cancer 2, 301-310, 2002).

Bislang bestand die Vorstellung, dass eine Virusinfektion, im Besonderen auch eine Infektion mit Influenza-Virus, über die Aktivierung der IKK den NF-kB Signalweg aktiviert, welcher wiederum entscheidend beteiligt ist an der durch diese Infektion erhöhten Expression von antiviral aktiven Proteinen wie beispielsweise Interferon (Chu et al., Immunity 11,721-731,1999). In Übereinstimmung mit dieser Vorstellung ist der Befund, dass die Influenza-Virus induzierte Aktivität des Interferon-β-Promotors stark reduziert ist in Zellen, welche eine transdominant negative Mutation von IKK2 oder von IkBalpha exprimieren (Wang et al., Virol 74,11566-11573,2000). Andererseits gibt es den diesen Vorstellungen und Befunden widersprechenden Hinweis, dass Acetylsalizylsäure, bekannt als Inhibitor der IKK , in der Zellkultur auch Influenza-Virus Infektionen hemmen kann, dieses jedoch erst ab Konzentrationen von 5-10 mM (entsprechend 0,9-1,8 mg/ml). Derartige Konzentrationen sind im Blut nach oraler Verabreichung von Acetylsalizylsäure ohne erhebliche Nebenwirkungen praktisch nicht erreichbar (Huang und Dietsch, New Engl J Med 319,797, 1988). Acetylsalizylsäure gilt als das toxischste aller derzeit frei verfügbare Analgetika mit der geringsten therapeutischen Breite (Jones, Am J Ther 9, 245-257,2002). Zwar spekulierten Huang und Dietsch, durch eine Verabreichung von Aerosol höhere und damit antiviral wirksame Konzentrationen lokal in den Atemwegen zu erzielen. Diese Empfehlungen wurden bislang jedoch nicht umgesetzt, da allein schon die bisher bekannten klinischen Nebenwirkungen nach oraler Verabreichung einer ansonsten als nicht-toxisch geltenden Dosis von 100mg Acetylsalizylsäure dazu führten, vor der Einnahme von Aspirin bei Virusgrippe besonders bei Kindern zu warnen (Gebrauchsinformation Z.Nr.14.252 der Bayer AG zum Acetylsalicylsäure (ASS)-Präparat Aspirin®) .

### Technisches Problem der Erfindung

Der Erfindung liegt das technische Problem zu Grunde, ein Testsystem zu schaffen, mit welchen sich verbesserte Wirksubstanzen zur Prophylaxe und/oder Behandlung von Viruserkrankungen finden lassen, sowie pharmazeutische Zusammensetzungen und Formulierungen für solche Indikationen anzugeben.

### Grundzüge der Erfindung und Ausführungsformen.

Die Erfindung beruht auf den überraschenden Erkenntnissen, dass i) Wirkstoffe, welche den zellulären NF-kB Signalübertragungsweg hemmen, die Vermehrung von Viren in einem Organismus hemmen können, ii) bei lokaler Verabreichung geringere Konzentrationen des erfindungsgemäß eingesetzten Wirkstoffes antiviral wirksam sind als aus den in vitro Daten ableitbar, iii) der erfindungsgemäß eingesetzte Wirkstoff Acetylsalizylsäure aerogen in Konzentrationen von 0.1 bis 4 mM verabreicht eine deutliche Hemmung der Influenza-Virusvermehrung in der Lunge und im Gesamtorganismus und einen systemischen Heileffekt ohne Beeinträchtigung des Allgemeinbefindens bewirkt, obwohl derartige Konzentrationen der Acetylsalizylsäure in vitro keine antivirale Wirksamkeit gezeigt haben. Höhere Dosen von Acetylsalizylsäure, beispielsweise Acetylsalizylsäure in Konzentrationen von 10 mM, waren vergleichsweise nicht stärker, noch höhere Dosen (bis 50 mM) deutlich geringer antiviral wirksam als die niedrigen, erfindungsgemäßen Konzentrationen. Zusätzlich erwiesen sich Konzentrationen ab 20mM nach aerogener Verabreichung als toxisch.

Gegenstand der vorliegenden Erfindung ist somit die Verwendung mindestens einer Wirksubstanz zur Herstellung einer pharmazeutischen Zusammensetzung zur Prophylaxe und/oder Therapie von mindestens einer Viruserkrankung, wobei die Wirksubstanz(en) auf mindestens einen Bestandteil des NF-kB Signalübertragungsweges hemmend einwirkt, sodass eine Virusvermehrung im Organismus gehemmt wird. Bestandteil des NF-kB Signalübertragungs- weges sind beispielsweise: Tumor necrosis factor receptor associated factor (TRAF), NF-kB inducing kinase (NIK), Mitogen-activated protein kinase kinase kinase 1 (MEKKK1), Mitogen-activated protein kinase kinase kinase 3 (MEKKK3), AKR mouse thymoma Kinase (AKT), TGFß activated kinase (TAK1), Inhibitor of NF-kB kinase alpha (IKKalpha), Inhibitor of NF-kB kinase beta (IKKbeta), NEMO, Inhibitor von kB (IkB), RELA (p65), C-REL, RELB, NF-kB1 (p105), NF-kB2 (p100), P50, P52.

Zu den erfindungsgemäß einsetzbaren Wirksubstanzen gehören beispielsweise: Inhibitoren einer Kinase des NF-kB Signalübertragungsweges [hierzu gehören nicht-steroidale anti-inflammatorische, die NF-kB Aktivierung inhibierende Substanzen, wie beispielsweise Phenylalkylsäurederivate wie beispielsweise Sulindac (Yamamoto et al., J Biol Chem 274,27307-27314, 1999; Berman et al Clin Cancer Res 8, 354-360, 2002) oder Derivate von Sulindac wie Sulindac- Sulfoxid, Sulidac- Sulfon, Sulindac- Sulfid, Benzylamid Sulindac-Analoga (Moon und Lerner, Cancer Research 62, 5711-5719,2002), Salicylsäurederivate wie beispielsweise Salicylsäure selber oder Acetylsalizylsäure (Yin et al., Nature 396, 77-80,1998) Salicylamid, Salacetamid, Ethenzamid, Diflunisal, Olsalazin oder Salazosulfapyridin, Curcumin (Oncogene 18, 6013-6020, 1999), Antioxidantien wie Pyrrolidine-dithiocarbamate (PDTC; Piette et al. Biol Chem 378, 1237-1245, 1997), Oxicame wie beispielsweise Piroxicam (Liu et al., J Biol Chem 13,---, 2002), Vitamin E und Derivate hiervon, wie beispielsweise Pentamethyl-hydroxychroman (PMC, Hattori et al., Biochem Mol Biol Int 35, 177-183, 1995), 17 beta Oestradiol und Derivate hiervon (Deshpande et al Am J Reprod Immunol 38, 46-54, 1997), Polyphenole des Tees wie beispielsweise (-)-epigallo-catechin-3-gallat (EGCG, Lin et al Biochem Pharmacol 58, 911915, 1999), Bay11-7182 Misra und Pizzo Arch Biochem Biophys 386, 227-232,2001)], Peptide, welche die Interaktion von mindestens zwei Komponenten des NF-kB Signalübertragungsweges inhibieren; hierzu gehören beispielsweise an NEMO bindende Peptide (May et al Science 289, 1550-1554, 2000), Inhibitoren des Proteosoms, wie beispielsweise PS-341 ( Tan und Waldmann, Cancer Res 62, 1083-1086, 2002; Adams Trends Mol Med 8, 49-54, 2002) und Lactacystin (Morise und Grisham J Clin Gastroenterol 27, 87-90,1998), Antisense-Oligonukleotide, welche sich spezifisch an die DNA-Sequenz oder m-RNA Sequenz kodierend für eine Komponente des NF-kB Signalübertragungsweges anlagern und deren Transkription oder Translation inhibieren, beispielsweise Antisense Nukleotidsequenzen spezifisch für p65 oder p50 ( Higgins et al PNAS-USA 90, 9901-9905,1993), Dominant negative Mutanten einer Komponente des NF-kB Signalüber- tragungsweges; dsOligonukleotide, die geeignet sind zur gezielten Degradierung der mRNAs einer Komponente des NF-kB Signalübertragungsweges durch die RNAi-Technologie entsprechend der Methode, wie von Tuschl et al (Genes Dev 13: 3191-3197, 1999) und von Zamore et al (Cell 101: 25-33, 2000) beschrieben, Antikörper oder Antikörperfragmente spezifisch für eine Komponente des NF-kB Signalübertragungsweges, oder Fusionsproteine, enthaltend mindestens ein Antikörperfragment, beispielsweise ein Fv-Fragment, welche mindestens eine Komponente des NF-kB Signalübertragungsweges inhibieren.

Wirksubstanz im Sinne der vorliegenden Erfindung ist eine Substanz, die in der Lage ist, auf mindestens eine Komponente des NF-kB Signalübertragungsweges direkt derart einzuwirken, dass eine Virusvermehrung im wesentlichen gehemmt ist. Weiterhin sind als Wirksubstanzen im Sinne dieser Erfindung Derivate dieser Wirksubstanzen zu verstehen, die beispielsweise durch enzymatische Spaltung in eine erfindungsgemäße aktive Wirksubstanz umgewandelt werden. Wirksubstanzen im Sinne der vorliegenden Erfindung sind außerdem Vorstufen von Wirksubstanzen, welche metabolisch in eine erfindungsgemäß aktive Substanz umgewandelt werden.

Bevorzugt erfolgt die Verwendung mindestens einer erfindungsgemäßen Wirksubstanz zur Prophylaxe oder Therapie einer Viruserkrankungen, die durch RNA- oder DNA-Viren, vorzugsweise Negativstrang-RNA Viren, beispielsweise Influenza-Viren, oder Borna-Viren, hervorgerufen werden. Hierzu wird die erfindungsgemäße Wirksubstanz systemisch oder lokal, beispielsweise dermal, nasal, aerogen, in eine Körperhöhle oder in ein Gewebe verabreicht.

Weiterhin bevorzugt ist die Verwendung von Acetylsalizylsäure zur Prophylaxe oder Therapie einer Influenza-Virus-Infektion, wobei die Acetylsalizylsäure nasal oder bronchial (aerogen) in Konzentrationen von vorzugsweise 0,1 bis 4 mM verabreicht wird. Die Gesamtdosis einer pro Tag beim Menschen sollte zweckmäßigerweise im Bereich von 0,1 bis 30 mg (nasal) bzw. von 0,1 bis 70 mg (bronchial) liegen. Je nach Anwendung kann die Untergrenze aber auch zwischen 0,1 und 20 mg bzw. 50 mg liegen. Je nach Anwendung kann aber auch die Obergrenze zwischen 1 mg bzw. 2 mg und den angegebenen Maximalwerten liegen. Eine Tagesdosis wird vorteilhafterweise in 1 bis 8 Gaben verabreicht, welche zweckmäßigerweise gleichmäßig über eine Wachdauer von 16 Stunden verteilt sind. Eine Behandlung erfolgt zweckmäßigerweise über einen Zeitraum von 1 bis 7 Tagen und länger. Die Erfindung umfaßt insofern auch galenisch hergerichtete Gabeeinheiten, wobei die in einer Gabeeinheit vorliegende Menge des Wirkstoffes gemäß vorstehenden Bereichen eines Behandlungsplanes unschwer errechenbar ist.

Eine weitere Ausführungsform der vorliegenden Erfindung betrifft ein Kombinationspräparat für die Prophylaxe und/oder Therapie einer Viruserkrankung, enthaltend mindestens zwei antiviral wirkende Wirksubstanzen, wobei mindestens eine antiviral wirkende Wirksubstanz mindestens eine Komponente des NF-kB Signalübertragungsweges derart hemmt, dass die Vermehrung des Virus in einem Organismus gehemmt wird. Vorzugsweise wird diese antiviral wirkende Wirksubstanz ausgewählt aus den bereits vorstehend aufgeführten erfindungsgemäßen Wirksubstanzen. Zu den weiteren antiviral wirkenden Wirksubstanzen in dem erfindungsgemäßen Kombinationspräparat gehören entweder mindestens eine andere erfindungsgemäße Wirksubstanz und/oder mindestens eine antvirale Wirksubstanz wie beispielsweise: Amantadin (1-Adamantanamin) und dessen Derivate, welches bzw. welche gegen ein Transmembranprotein von einigen Influenza-A Viren gerichtet ist bzw. sind, wie beispielsweise das Rimantadine, Therapeutika für Influenzainfektionen, die das Influenza-virale Oberflächenprotein Neuraminidase hemmen. Hierzu gehört beispielsweise Relanza, Inhibitoren des Raf-MEK-ERK- Signalübertragungsweges wie zum Beispiel U0126 oder weiterer Inhibitoren, wie sie in der PCT/DE 01/01292 beschrieben wurden, Inhibitoren des MEKK/SEK-Signalübertragungsweges oder der Komponenten weiterer Signalübertragungswege wie sie in der DE 10138 912 beschrieben wurden und synthetische Nucleosidanaloga wie beispielweise 3-Deaza adenosin und Ribavirin.

Das Kombinationspräparat kann in Form eines Gemisches oder als einzelne Komponenten zur gleichzeitigen oder zeitlich unterschiedlichen Anwendung an gleichen oder unterschiedlichen Orten systemisch oder lokal angewendet werden. Es gelten die vorstehend zu Darreichungsformen getroffenen Ausführungen analog.

Die Verabreichung des Kombinationspräparates kann als Mischung der Wirksubstanzen erfolgen. Die Wirksubstanzen können pro Verabreichung jedoch auch getrennt voneinander an dem gleichen Ort, beispielsweise systemisch durch intravenöse Injektion oder lokal, beispielsweise durch nasale, aerogene oder dermale Verabreichung oder durch Injektion in ein Gewebe oder auch an voneinander getrennten Orten, gleichzeitig oder auch zu unterschiedlichen Zeitpunkten innerhalb eines Zeitraumes, in welchem die zuerst verabreichte Substanz noch Wirkung zeigt, beispielsweise in einem Zeitraum von drei Tagen, verabreicht werden.

Eine besondere Form der Verabreichung der erfindungsgemäßen Wirksubstanz im Sinne der Erfindung ist die aerogene, das heißt nasale oder bronchiale Verabreichung der Wirksubstanz zur Prophylaxe oder Therapie derjenigen Virusinfektionen, welche aerogen infizieren. Hierzu werden galenische Hilfsmittel und Zerstäuber der Wirksubstanz verwendet, wie sie dem Fachmann hinreichend bekannt sind.

Eine weitere Ausführungsform der vorliegenden Erfindung betrifft ein Testsystem zum Auffinden von Wirksubstanzen, welche mindestens eine Komponente des NF-kB Signalübertragungsweges derart hemmen, dass eine Virusvermehrung gehemmt wird, enthaltend a. mindestens eine mit mindestens einem Virus infizierbare Zelle, die den NF-kB Signalübertragungsweg enthält und mindestens einen die Zellen infizierenden Virus oder b. mindestens eine mit mindestens einem Virus infizierte Zelle, bei welcher mindestens eine Komponente des NF-kB Signalübertragungsweges fehlt oder defekt mutiert ist.

Zellen im Sinne der vorliegenden Erfindung sind Zellen aus unterschiedlichen Organen und Geweben, beispielsweise Zellen der Blut- und Lymphgefäße, Zellen, die Körperhöhlen auskleiden. Ebenfalls umfasst sind Zellkulturen, besonders solche, welche von Zellbanken, beispielsweise der ATCC, zu erwerben sind, insbesonders permissive, eukaryotische Zellkulturen, beispielsweise A549, 293, 293T und 293T7 (*Homo sapiens*)*,* B82, NIH 3T3, L929 aus *Mus musculus,* BHK aus *Cricetus cricetus,* CHO aus *Cricetulus griseus,* MDCK aus *Canis familiaris,* Vero, COS-1 und COS-7 aus *Cercopithecus aethiops,* sowie primäre Embryo-Fibroblasten aus *Gallus gallus* (CEF cells).

Beispielsweise wird in dem erfindungsgemäßen Testsystem zum Auffinden von Wirksubstanzen durch Zugabe von Substanzen, vorzugsweise in Konzentrationen von 0.001 µM bis 100 µM, und Viren in einer Partikelzahl, welche die ausgewählte Zelle infizieren können, geprüft, ob eine Substanz in der Lage ist, die Virusvermehrung zu hemmen, ohne die Zelle zu schädigen.

Vorzugsweise handelt es sich bei dem in dem erfindungsgemäßen Testsystem verwendeten Virus um ein RNA- oder DNA-Virus, vorzugsweise um ein Influenza-Virus.

In einer bevorzugten Ausführungsform enthält die Zelle a) des erfindungsgemäßen Testsystems mindestens eine überexprimierte Komponente des NF-kB Signalübertragungsweges, auch in Form von konstitutiv aktiven Mutanten dieser Komponenten, insbesondere durch Einführung eines oder mehrerer diese Komponente kodierenden Gens bzw. Gene. Durch diese Überexpression werden Substanzen entdeckt, welche diese Komponente sowohl stark inhibieren wie auch intrazellulär zur Inhibition der überexprimierten Komponente eine hohe Konzentration erreichen können. Zur Kontrolle ist in einer Zelle b) des erfindungsgemäßen Testsystems die Expression für mindestens eine Komponente des NF-kB Signalübertragungsweges inhibiert, beispielsweise durch die Einführung einer antisense DNA oder einer antisense RNA oder durch die Einführung mindestens eines Gens kodierend für mindestens eine dominantnegative Mutante mindestens einer Komponente des NF-kB Signalübertragungsweges.
Eine weitere Ausführungsform der vorliegenden Erfindung betrifft ein Verfahren zum Auffinden von mindestens einer erfindungsgemäßen Wirksubstanz zur Prophylaxe und/oder Therapie von Viruserkrankungen, welche die Vermehrung von Viren bei Viruserkrankungen hemmt bzw. hemmen, enthaltend folgende Schritte: a. Inkontaktbringen mindestens eines erfindungsgemäßen Testsystems mit mindestens einer potentiellen Wirksubstanz, und b. Bestimmung der Auswirkung auf die Virusvermehrung.

Inkontaktbringen im Sinne dieser Erfindung kann beispielsweise durch Zugabe der Wirksubstanzen in das Nährmedium einer Zellkultur oder durch lokale oder systemische Verabreichung der Wirksubstanzen in einen Organismus erfolgen.

Inkontaktbringen im Sinne der vorliegenden Erfindung umfasst ebenfalls die nach dem Stand der Technik üblichen Verfahren, die das Einführen von Substanzen in intakte Zellen erlauben, beispielsweise Infektion, Transduktion, Transfektion und/oder Transformation und weitere dem Fachmann bekannte Methoden. Diese Verfahren sind insbesondere bevorzugt, wenn es sich bei der Substanz um Viren, nackte Nukleinsäuren, beispielsweise antisense DNA und/oder antisense RNA, Viroide, Virosomen und/oder Liposomen handelt, wobei Virosomen und Liposomen ebenfalls geeignet sind, neben einem Nukleinsäuremolekül weitere Wirksubstanzen in die Zelle zu bringen.

Das Bestimmen der Auswirkung auf die Virusvermehrung erfolgt beispielsweise durch Plaque-Assays oder durch Bestimmung der HA-Units zum Vergleich des Virustiters behandelter und nicht behandelter infizierter Zellen.

Eine weitere bevorzugte Ausführungsform der vorliegenden Erfindung betrifft ein Verfahren zur Herstellung eines Arzneimittels zur Prophylaxe und/oder Therapie von mindestens einer Viruserkrankung, welche die Vermehrung von Viren bei Viruserkrankungen hemmt bzw. hemmen, enthaltend folgende Schritte: a. Durchführen eines erfindungsgemäßen Testsystems und b. Versetzen des/der aufgefundenen und in physiologisch wirksamer Dosis dosierten Wirksubstanz/en mit mindestens einem Hilfs-und/oder Zusatzstoff und definierter galenischer Herrichtung.

Vorzugsweise wird die Wirksubstanz gemäß vorliegender Erfindung für die lokale oder systemische Verabreichung in einen Organismus mit Hilfe der dem Fachmann geläufigen Methoden und Hilfs-und/oder Zusatzstoffen zu einem Arzneimittel zubereitet.

Geeignete Hilfs- und Zusatzstoffe, die beispielsweise der Stabilisierung oder Konservierung des Arzneimittels oder Diagnostikums dienen, sind dem Fachmann allgemein bekannt (siehe z. B. Sucker H et al., (1991) Pharmazeutische Technologie, 2. Auflage, Georg Thieme Verlag, Stuttgart). Beispiele für solche Hilfs- und/oder Zusatzstoffe sind physiologische Kochsalzlösungen, Ringer-Dextrose, Dextrose, Ringer-Laktat, entmineralisiertes Wasser, Stabilisatoren, Antioxidantien, Komplexbildner, antimikrobielle Verbindungen, Proteinaseinhibitoren und/oder inerte Gase.

Die lokale Verabreichung kann beispielsweise auf die Haut, auf die Schleimhaut, in eine Körperhöhle, in ein Organ, in ein Gelenk oder in das Binde- oder Stützgewebe nasal oder aerogen erfolgen. Die systemische Verabreichung erfolgt vorzugsweise in den Blutkreislauf, in die Peritonealhöhle oder in die Bauchhöhle.

Die Arzneimittelzubereitüng-enthaltend die erfindungsgemäße Wirksubstanz richtet sich nach der Art der Wirksubstanz und der Art ihrer Verabreichung und kann beispielsweise eine Lösung, eine Suspension, eine Salbe, ein Puder, eine Sprayform oder eine andere Inhalationszubereitung darstellen. Vorzugsweise werden Nukleotidsequenzen mit dem Fachmann bekannten Methoden in einen viralen Vektor oder ein Plasmid eingefügt und mit Hilfsstoffen für die Zelltransfektion versetzt. Zu diesen Hilfsstoffen gehören beispielsweise kationische Polymere oder kationische Lipide. Antisense-Oligonukleotide werden mit den dem Fachmann geläufigen Methoden derivatisiert, um sie vor dem enzymatischen Abbau durch DNAsen oder RNAsen zu schützen.

Die Wirksubstanz gemäß der Erfindung kann in Form eines Salzes, Esters, Amids oder als eine Vorstufe vorliegen, wobei vorzugsweise nur Modifikationen der Wirksubstanz verwendet werden, die keine übermäßige Toxizität, Irritationen oder allergische Reaktionen am Patienten auslösen.

Die Wirksubstanz wird unter sterilen Bedingungen, mit einem physiologisch akzeptablen Trägerstoff und möglichen Preservativen, Puffern oder Treibmitteln, je nach Bedarf, vermischt. Derartige Trägerstoffe für Arzneimittelzubereitungen sind dem Fachmann geläufig.

Bevorzugt wird die erfindungsgemäße Wirksubstanz in einer einmaligen Dosis, besonders bevorzugt in mehreren Dosen verabreicht, wobei die einzelnen Dosen die maximal tolerable Dosis (MTD) der jeweiligen Wirksubstanz für den Menschen nicht übersteigt. Vorzugsweise wird eine Dosis gewählt, welche die Hälfte der MTD beträgt. Die Tagesdosis kann einmalig am Tag oder in mehreren Teilportionen aufgeteilt über den Tag hinweg, vorzugsweise in etwa gleichen Zeitabständen verabreicht werden.

Gemäß der vorliegenden Erfindung kann die Verabreichung entweder lokal oder systemisch, nur an einem Tag oder über mehrere Tage täglich oder an jedem zweiten oder dritten Tag über mehrere Wochen erfolgen, bis eine therapeutische Wirkung sichtbar wird.

### Beispiel 1: Testsystem zur Auffindung von antiviralen Wirkstoffen

Mit Hilfe retroviraler Transduktion der humanen Lungenepithelzellen A549, der caninen Nierenepitehlzellen MDCK und der Affenzellen Vero wurden Zelllinien hergestellt, welche stabil entweder eine dominant negative Form der IKK (IKK KD) oder eine dominant-interferierende mutierte Form des Inhibitors von NF-kB, mIkB, exprimieren. Darüber hinaus wurden ebenfalls entsprechende Linien generiert, welche eine aktive Form der IKK (IKK EE) exprimieren. Sowohl die Konstrukte als auch Ihre Wirksamkeit auf die NF-kB vermittelte Genexpression in Zellen wurden bereits beschrieben (Denk et al J Biol Chem 276, 28451-28458, 2001).

Zur Generierung der stabilen Zelllinen wurde die entsprechenden cDNAs für IKK KD, mIkB und IKK EE in sense-Orientierung in den retroviralen Expressionsvektor pCFG5 IEGZ (Kuss et al. Eur J Immunol 29,3077-3088,1999) einkloniert. Neben der Boten-RNA des 'gene of interest' kodiert die Vektor DNA noch für die Boten-RNA des "green fluorescent protein" (GFP), welches während der Proteinsynthese ausgehend von einer internen Ribosomenbindestelle exprimiert wird. Dies erlaubt die Identifizierung stabil transduzierter Zellen in der Durchflusszytometrie. Darüber hinaus vermittelt der Vektor eine Resistenz gegen das Antibiotikum Zeocin. Die verschiedenen Expressionskonstrukte sowie der Leervektor wurden mit Hilfe der Calciumphosphatprezipitationsmethode in die virusproduzierende Zelline ØNX (Grignani et al., Cancer Res 58,14-19,1998) transfiziert (Denk et al., J Biol Chem 276, 28451-28458, 2001). Die Transfektionseffizienz wurde nach 24h anhand der GFP Expression kontrolliert und war in der Größenordnung von 70-80%. Die Zellen wurden darauf hin für ca. 2 Wochen mit 1mg/ml Zeocin im Medium selektioniert.

Zur Infektion der verschiedenen Zielzellen (A549, MDCK, Vero) mit den rekombinanten Retroviren wurden die retrovirushaltigen Mediumüberstände der virusproduzierenden Zellinien filtriert, mit 5 µg/ml Polybrene (Sigma) versetzt und auf frische Zellen gegeben. Die Infektion erfolgte während 2mal 3-stündiger Zentrifugation (1000xg) an zwei aufeinander folgenden Tagen. Stabil transduzierte Zellen wurden 24h nach Infektion für zwei weitere Wochen mit 400-600 µg/ml Zeozin im Mediumüberstand selektioniert. Nach der so erfolgten Generierung der stabilen Zellinien wurden sowohl diese als auch Wildtyp Influenza A Virus wie im folgenden beschrieben infiziert und die Virustiter von Zellen, welche stabil den Vektor, IKK KD, mIkB und IKK EE trugen im Vergleich zum Titer aus dem Überstand von Wildtyp Zellen bestimmt.

Parallel wurden MDCK Zellen mit Hilfe des Transfektionsreagenz Lipofectamine 2000 (Life Technologies) nach Standardmethoden (Ludwig et al., J Biol Chem 276,10990-10998, 2001) mit den gleichen Konstrukten transient transfiziert. Die Transfektionseffizienzen lagen bei >60%. 24h nach Transfektion erfolgte wie bei den stabilen Linien Infektion mit dem Influenza A Virusstamm fowl plaque virus (FPV) mit einer Multiplizität der Infektion von 1 (M.O.I.=1). Weitere 24h nach Infektion wurden die Titer der neu gebildeten Viren im Zellkulturüberstand in Standard Plaque-Assays auf MDCK Zellen überprüft. Verglichen wurde auch hier der Virustiter von Influenza A Virus-infizierten Zellen, welche entweder mit dem Leervektor oder Konstrukten welche IKK KD, mIkB und IKK EE exprimierten, transfiziert waren.

Es zeigten sich die folgenden Ergebnisse. Der Vergleich der Virustiter von Influenza A Virus-infizierten MDCK Zellen, welche zuvor entweder mit dem Leervektor oder einem Konstrukt, welches IKK KD oder mIkB exprimierte, transfiziert worden waren, zeigte, dass in IKK KD oder mIkB exprimierenden Zellen die Vermehrung der Viren nach 24h zu 50%-70% gehemmt war. Entsprechend fand man in Zellen, welche die aktive Form von IKK, IKK EE exprimierten, ein Steigerung der Virusvermehrung. Diese Ergebnisse waren in mehreren unabhängigen Ansätzen reproduzierbar. Entsprechende Ergebnisse konnten auch in den stabilen A549, MDCK und Vero Zelllinien erhalten werden, welche entweder IKK KD, mIkB oder IKK EE exprimierten, wobei die größten Effekte in stabil transfizierten A549 Zellen beobachtet wurden. Hier führte eine Hemmung der NF-kB Aktivierung durch IKK KD oder mIkB zu einer bis zu 10-fachen Reduktion der Virustiter, während konstitutive Aktivierung des Signalwegs durch stabile Expression von IKK EE die Virusausbeute um das zehnfache verstärkte. Diese Befunde zeigen, dass Aktivierung von IKK und NF-kB essentiell für die Influenza Virus Vermehrung ist und die spezifische Inhibition des IKK/NF-kB Signalmoduls s zu einer signifikanten Reduktion der Virusproduktion führt.

### Beispiel 2: Hemmung der viralen NF-kB Aktivierung und Reduktion der Influenza Virus in vitro

### 2.1: Acetylsalizylsäure (ASS)

Salicylate, wie ASS oder Sulfosalazine finden weitgehende klinische Anwendung als Schmerzmittel und antiinflammatorische Agenzien. Neuere Publikationen zeigen, dass diese Substanzen direkte und effektive Inhibitoren der IKK sind (Yin et al., Nature 396, 77-80, 1998; Weber et al., Gastroenterology 119,1209-1218, 2000) wie auch in vitro die Vermehrung von Influenzaviren hemmen können (Huang und Dietsch, New Engl J Med 319,797,1988). Als Positivkontrolle diente somit ASS. A549 Lungenepithelzellen wurden mit ansteigenden Konzentrationen von ASS im Bereich 0,01 mM-5mM behandelt. Diese Konzentrationen verblieben über das gesamte Experiment im Kulturmedium. Eine Stunde nach Behandlung erfolgte Infektion mit dem Influenza A Virusstamm fowl plaque virus (FPV) mit einer Multiplizität der Infektion von 1 (M.O.I.=1). Weitere 24h nach Infektion wurden die Titer der neu gebildeten Viren im Zellkulturüberstand in Standard Plaque-Assays auf MDCK Zellen überprüft.

In einem zweiten Ansatz wurden MDCK Zellen ein Stunde vor Infektion bzw. zwei und vier Stunden nach Infektion mit 5 mM ASS behandelt. Infektion und Nachweis der neu-gebildeten Viren erfolgte wie oben.

Es zeigten sich die folgenden Ergebnisse. Nach Vergleich der Virustiter aus A549 Kulturüberständen 24h p.i. von nicht ASS-behandelten mit ASS-behandelten Zellen ergab sich ein konzentrationsabhängige Inhibition der Virusvermehrung, die jedoch erst deutlich war bei 5mM und hier 2 log Stufen umfasste. Eine entsprechende hemmende Wirkung (> 2 log Stufen) von 5mM ASS konnte auch bei infizierten MDCK Zellen beobachtet werden, wobei hier bei Zugabe von ASS 4h nach Infektion immer noch eine Reduktion der Virustiter um das 10-fache bewirkte.

### 2.2: Pyrrolidine-dithiocarbamate (PDTC)

Antioxidantien wie Pyrrolidine-dithiocarbamate (PDTC) sind hinlänglich als Inhibitoren von NF-kB Aktivierung bekannt (Übersicht in: Piette et al., Biol Chem 378, 1237-1245, 1997). Daher wurde untersucht, ob auch diese Substanzklasse hemmend auf die Influenza Virus Vermehrung wirken kann. A549 Zellen wurden eine Stunde vor Infektion mit PDTC in Konzentrationen von 3-24 micromolar behandelt. Infektion und Nachweis der neu-gebildeten Viren 24h p.i. erfolgte wie für ASS beschrieben in An- bzw. Abwesenheit von PDTC über das gesamte Experiment.

Es zeigten sich die folgenden Ergebnisse. Auch PDTC Behandlung führte zu einer konzentrationsabhängigen Inhibition der Virustiter in A549 Zellen bis zu hin zu einer etwa 10-fachen Reduktion bei Gabe der maximal eingesetzten Konzentration von 24 micromolar. Diese Daten zeigen, dass die IKK und NF-kB hemmenden Agenzien ASS und PDTC, analog der Wirkung dominant-negativer Mutanten aus dem NF-kB Signalmodul, einen signifikant hemmenden Effekt auf die Influenza Virus-Vermehrung in Zellkultur haben.

### Beispiel 3: Wirkung von ASS auf die Influenzainfektion in der Maus

### 3.1: i.p./parenterale Verabreichung

C57 B1/6 Mäuse wurden mit 5000 pfu/20µl Influenza-Virus (Fowl Plague Virus ,FPV) nasal infiziert. 30 min vor Infektion wurden 500µl ASS (50mM = 9mg/ml PBS, Sigma-Aldrich Steinheim) i.p. injiziert, nachfolgend wurde ASS (50 mM = 9mg/ml) kontinuierlich im Trinkwasser verabreicht. Zur Kontrolle wurde PBS injiziert bzw, verabreicht. Körpergewicht, Todesrate und Überlebensdauer wurde bestimmt. Pro Gruppe wurden 30 Mäuse behandelt.

Es zeigten sich die folgenden Ergebnisse. Während in der Kontrollgruppe alle Tiere an Influenza starben, überlebten in der ASS Gruppe etwa 20% der Tiere die Infektion. Des weiteren war die mittlere Überlebenszeit der verstorbenen Tiere im Vergleich zur Kontrollgruppe deutlich verlängert. ASS in Konzentrationen von 50 mM verabreicht führte jedoch zu einer deutlichen Verminderung des Körpergewichtes aufgrund der toxischen Dosis.

### 3.2: aerogene Verabreichung

C57 B1/6 Mäuse wurden mit 5000 pfu/20µl bzw mit 10.000 pfu/20 ml Influenza-Virus (Fowl Plague Virus ,FPV) nasal infiziert.
Bei Behandlungsbeginn am Tag 0 wurde 30 min nach der Infektion in die Mäuse unter Betäubung (i.p. Injektion von 300µl Ketamin/Rompun; Serum Werk Bernburg; Bayer Ag Leverkusen) ein Tracheotubus eingeführt und mit Hilfe eines Nebulizers (Hugo Sachs Elektronik-Harvard App.GmbH March-Hugstetten) Aerosol (Verabreichung von 600 µl) enthaltend 2mM (= 0,36mg/ml PBS), 10 mM, 20mM oder 50 mM ASS (Sigma-Aldrich Steinheim) oder zur Kontrolle nur PBS verabreicht. Diese aerogene Verabreichung der Prüfsubstanzen wurde in einigen Gruppen an den Tagen 1, 2, 3 oder 1, 2, 5 und 6 wiederholt. In weiteren Gruppen wurde die Behandlung mit ASS an den Tagen 3, 4, 5, 6 nach Infektion durchgeführt. Pro Gruppe wurden 5-6 Tiere behandelt. Körpergewicht, Todesrate und Überlebensdauer wurde bestimmt. In einem weiteren Versuch wurden nach Behandlung mit ASS am Tag 1 am Tag 3 die Tiere getötet und die Viruskonzentration in dem Lungengewebe bestimmt.

Es zeigten sich die folgenden Ergebnisse. Geringere Dosen von ASS reduzierten die Viren in der Lunge deutlicher als hohe Dosen. ASS erwies sich ab 20 mM als toxisch (Abnahme des Körpergewichtes). Während in der Kontrollgruppe alle Tiere an Influenza starben, überlebten in den Gruppen behandelt mit niedrigen nichttoxischen Dosen (2mM) von ASS etwa 40% die Infektion. Des weiteren war die mittlere Überlebenszeit der verstorbenen Tiere nach ASS im Vergleich zur Kontrollgruppe deutlich verlängert.

Die Ergebnisse in vivo Experimente gemäß der Beispiele 3.1 und 3.2 zeigen, dass die einfache oder mehrfache (bis zu 3x) tägliche aerogene Verabreichung von ASS in niedriger nichtoxischer Dosis, i.e. in Dosen von 0,1 mg/kg bis 300 mg/kg Körpergewicht, insbesondere 10 mg/kg bis 100 mg/kg, vorzugsweise 20 mg/kg bis 50 mg/kg, beispielsweise 30 mg/kg, zu einer deutlichen therapeutischen Wirkung gegen eine tödliche durch nasale Verabreichung des Influenza-Virus induzierte Erkrankung führt. Dabei sollte die Formulierung so gewählt sein, dass die aerogen zu verabreichende pharmazeutische Zusammensetzung ASS in Konzentrationen unterhalb 2 mM, vorzugsweise 0,01 mM bis 1,99 mM, höchstvorzugsweise 0,1 mM oder 0,5 mM bis 1,5 mM, beispielsweise 1 mM liegt. Die Flüssigphasenmenge an Aerosol ist dabei entsprechend der oben angegebenen Tagesdosen unter Berücksichtigung der eingesetzten Konzentration in der Flüssigphase auszurechnen und einzurichten. Letzteres läßt sich beispielsweise mit üblichen Zerstäubungsvorrichtungen erreichen, welche definierte Mengen einer Lösung zu einem Aerosol versprühen.

## Patentansprüche

1. Pharmazeutische Zusammensetzung zur nasalen, bronchialen oder aerogenen Verabreichung enthaltend Acetylsalicylsäure (ASS) für die Verwendung bei der Prophylaxe und/oder Therapie einer Influenzainfektion.

2. Pharmazeutische Zusammensetzung gemäß Anspruch 1 zur bronchialen Darreichung als Aerosol.

3. Pharmazeutische Zusammensetzung gemäß Anspruch 1 oder 2, wobei die Zusammensetzung eine weitere antivirale Wirksubstanz enthält.

4. Pharmazeutische Zubereitung gemäß Anspruch 1 oder 2, wobei der Wirkstoff in Dosen von 0,1 mg/kg bis 300 mg/kg Körpergewicht verabreicht wird.

5. Pharmazeutische Zubereitung gemäß Anspruch 4, wobei der Wirkstoff in Dosen von 20 bis 50 mg verabreicht wird.

6. Pharmazeutische Zusammensetzung nach einem der Ansprüche 1 bis 3, wobei der Wirkstoff aerogen in Konzentrationen von 0,1 bis 4 mM verabreicht wird.
